# EUROPEAN PATENT APPLICATION

(11) **EP 1 537 854 A1**
(43) Date of publication of application: **08.06.2005**
(21) Application number: 03293000.0
(22) Date of filing: 01.12.2003
(51) Int. Cl.: A61K 7/42, A61K 7/00

(54) **High lipid content sprayable emulsions**

(71) Applicant: Johnson & Johnson Consumer France SAS, 92787 Issy les Moulineaux Cedex 9 (FR)
(72) Inventor: Brillouet, Anne-Sophie, 27400 Louviers (FR); Tischenbach, Isabelle, 27400 Louviers (FR)
(74) Representative: Weber-Bruls, Dorothée, Dr.

(57) **Abstract**

This invention relates to sprayable oil-in-water emulsions with at least 15% by weight of oil phase.

## Description

### Field of the Invention

This invention relates to sprayable oil-in-water emulsions with a high content of oil phase.

### Background of the Invention

Sprays are attractive as a vehicle to apply liquid formulations on the skin such as in skin care and cosmetic applications. Sprays are convenient to use and typically allow a more controlled and even application of the formulation to the skin. Formulations for use on the skin often are emulsion based, usually in the form of oil-in-water (O/W) emulsions, allowing the presence of both lipophilic and hydrophilic components in one formulation. In a number of instances an emulsion-based formulation requires a relatively high amount of lipophilic components. This for example is the case where the formulation needs to have high amounts of lipophilic actives, which in turn require the presence of relatively high amounts of liquid oily components that are needed to build a liquid oil phase. However, emulsions with high lipid phase content need relatively high amounts of emulsifier. This in turn results in an increase of viscosity having the consequence that such formulations cannot be sprayed.

Also sunscreen formulations are frequently formulated as oil-in-water emulsions. Sunscreen formulations having a high SPF (Sun Protection Factor) are nowadays more and more wanted by consumers because of increased awareness of skin damage and of the long term effects of exposure of the skin to sunlight. Sunscreen formulations with SPF values as high as SPF 50 and even higher are recommended for use on sensitive skin types and for children. Such high SPF sunscreen formulations require the presence of large amounts of UV filters. The largest choice of UV filters is amongst the lipophilic filters, which also are the most effective and therefore are widely used in sunscreen formulations. The choice of hydrophilic UV filters is limited and so is their effectiveness so that increasing the amount of this type of filters is not sufficient to reach high SPF value and moreover usually gives rise to white staining upon usage. Inorganic pigments are another type of filters, the amounts of which could also be increased, but this leads to problems of stability of the formulations and again of white staining. Consequently sunscreen formulations with high SPF values can only be obtained by adding larger amounts of lipophilic filters. This poses a special challenge to sprayable formulations since increasing the amount of lipophilic components in the formulations requires more emulsifier, resulting in increased viscosity.

WO-03/013455 relates to light protecting cosmetic or dermatological emulsions, which contain: (a) at least one benzotriazole derivative and (b) at least one grade of latex particles having a mean particle size of 100 to 400 nm.

EP-A-1 206 933 relates to compositions containing caprylyl glycol or an analogue thereof, and a preservative agent. It further relates to the use of caprylyl glycol or an analogue thereof to increase the activity of preservative agents. EP-A-1 238 651 additionally relates to chemical compositions containing caprylyl glycol or an analogue thereof, and a particular combination of preservative agents. It further relates to the use of caprylyl glycol or an analog thereof to increase the activity of preservative agents, more in particular of a combination of iodopropynyl butyl carbamate and phenoxyethanol.

EP-A-1 005 851 discloses cosmetic or dermatological compositions useful for treating Malassezia spp. infections, especially dandruff, comprising an alkynyl carbamate derivative and polyol.

Hence there is a need to provide sprayable formulations that have a high content of lipophilic components. In particular there is a need for sprayable formulations that contain relatively high amounts of lipophilic ingredients, in particular of lipophilic active ingredients. There is a more specific need to provide sprayable sunscreen formulations that have a high SPF value. Providing such formulations is an object of this invention.

### Summary of the invention

Thus in one aspect this invention concerns a sprayable oil-in-water emulsion comprising at least 15 % of oil phase, wherein the formulation contains at least 0.1 % of an alkylglycol.

In a further aspect the invention concerns a sprayable oil-in-water emulsion comprising at least 5 % of lipophilic active ingredients and wherein the emulsion contains at least 15 % of oil phase, wherein the formulation contains at least 0.1 % of an alkylglycol.

In a further aspect the invention concerns a sprayable oil-in-water emulsion which is a sunscreen formulation comprising at least 5 % of lipophilic UV filters, wherein the emulsion contains at least 15 % of an oil phase, and wherein the formulation contains at least 0.3 % of an alkylglycol. The sunscreen formulations of the invention may have a high SPF value, e.g. an SPF ≥ 40, more preferably ≥50, or even ≥60.

Particular embodiments of this invention are those emulsions, including any emulsions that contain active ingredients which can be UV filters or other active ingredients, that contain at least 20%, or more in particular at least 25 %, or further in particular at least 30 % of lipid phase.

Other particular embodiments of this invention are those emulsions, including any emulsions that contain active ingredients which can be UV filters or other active ingredients, that contain at least 0.3 % of alkyl glycol, more in particular at least 0.3 % of alkyl glycol.

In another aspect this invention concerns sprayable oil-in-water emulsions wherein the emulsion contains from 15 % to 40%, in particular from 20 to 30 % of an oil phase, and further comprising from 0.1 to 10%, in particular from 0.2 to 5%, more in particular from 0.3 to 3% of an alkylglycol, preferably caprylyl glycol.

In another aspect this invention concerns sprayable oil-in-water emulsions, wherein the emulsion contains from 15 % to 40%, in particular from 20 to 30 % of an oil phase, comprising :
(a) from 5 to 30 %, in particular from 10 to 30%, more in particular from 10 to 30%, further in particular from 10 to 20% of one or more lipophilic active ingredients, which active ingredients are included in the oil phase;
(b) from 0.1 to 10%, in particular from 0.2 to 5%, more in particular from 0.3 to 3% of an alkylglycol, preferably caprylyl glycol.

In another aspect, this invention concerns sprayable oil-in-water emulsion which are sunscreen formulations, wherein the emulsion contains from 15 % to 40%, in particular from 20 to 30 % of an oil phase, comprising:
(a) from 5 to 30 %, in particular from 10 to 30%, more in particular from 10 to 30%, further in particular from 10 to 20% of lipophilic UV filters, which filters are included in the oil phase;
(b) from 0.1 to 10%, in particular from 0.2 to 5%, more in particular from 0.3 to 3% of an alkylglycol, preferably caprylyl glycol.

A preferred alkyl glycol is caprylyl glycol, also referred to as 1,2-octanediol.

### Detailed description of the invention

As used in this specification and claims, any % is weight by weight, unless indicated otherwise. The symbol "≥" means greater or equal to.

The term 'sprayable' means that the composition can be applied by a standard spraying device used in consumer products. In particular, sprayable emulsions have a viscosity of 100 to 2000 cps in particular from 100 to 1000 cps, measured with a spindle rotation rheometer at a constant shear rate of 60 s⁻¹, equipped with a spindle appropriate for these viscosity ranges.

The alkyl glycol for use in the emulsions of the invention in particular can be C₆₋₁₄ alkyl glycols, more in particular C₈₋₁₂ alkyl glycols, further in particular C₈ alkyl glycols, preferably caprylyl glycol, which is also referred to as 1,2-octanediol. Examples of alkyl glycols comprise 2,3-octanediol, 1,2-nonanediol, 1,2-decanediol, 1,2-dodecanediol, 1,2-heptanediol, 1,2-hexanediol, 3,4-octanediol and the like. Of particular interest are those alkyl glycols wherein one hydroxyl group is substituted at an end carbon and the other on the carbon atom adjacent thereto.

The term 'oil phase' covers the sum of all lipophilic components. These may comprise oils, fats, waxes, lipophilic active ingredients, lipophilic polymers, etc.

### Oil Phase

The oil-in-water emulsions of the present invention contain an oil phase that may comprise suitable oils which are skin-compatible components or component mixtures that are non water-mixable such as, for example, silicone oils, natural oils, fatty acid esters, mono-, di- or triglycerides, or other oils, or mixtures thereof. Preferably, the oils are liquid at ambient temperature, in particular are liquid at 25 °C. They can contain certain amounts of solid lipid components (e.g. fats or waxes) as long as the complete oily mixture is liquid at ambient temperature or at the temperature mentioned above.

Oils, which can be incorporated in the oil phase comprise natural oils, or natural oil derivatives, in particular of vegetable origin. Examples are almond oil, soybean oil, sunflower oil, safflower oil, corn oil, canola oil, borage oil, evening primrose oil, grapeseed oil, wheat germ oil, avocado oil, jojoba oil, kernel oil, sesame oil, walnut oil, linseed oil, palm oil, olive oil, macadamia oil, castor oil, rapeseed oil, peanut oil, coconut oil, and turnip seed oil.

The oil phase in the emulsions may also contain mono-, di or triglycerides, including mixtures thereof. These can be the natural oils mentioned above but may also comprise oil components isolated from natural oils, for example triglycerides or triglyceride mixtures isolated from natural oils, or mono-, di or triglycerides having been prepared chemically. The mono-, di or triglycerides may be derived from saturated or unsaturated, linear or branch chained, substituted or unsubstituted fatty acids or fatty acid mixtures. In particular such glycerides comprise the mono-, di- or tri-C₆₋₃₀ fatty acid glycerides, more in particular the mono-, di- or tri-C₈₋₂₄ fatty acid glycerides, specifically the mono-, di-or tri-C₁₂₋₂₂ fatty acid glycerides. Also included are mixed glyceride esters, i.e. di- or triglyceride esters with two or three different fatty acids. Mixtures of mono-, di- and triglycerides include technical mixtures derived from fractions of fatty acids obtained by hydrolysis from fractions of oils or fats. An example of the latter is a technical mixture of C₁₂₋₁₈ fatty acid mono-, di- and triglycerides.

Examples of glyceride oils are mono-, di-or triglycerides derived from palmitic, palmic, oleic, lauric, myristic, stearic, hydroxystearic, behenic acid, or mixtures thereof.

Of particular interest are the trigycerides, including triglyceride mixtures, and also including mixed esters, i.e. tri-esters of glycerine with different fatty acids. Examples are glyceryl tristearate, glyceryl trioleate, glyceryl tripalmitate, glyceryl trimyristate, glyceryl tribehenate, glyceryl trilaurate.

Interesting mono- or diglycerides are the mono- or di-C₁₂₋₂₄ fatty acid glycerides, specifically the mono- or di-C₁₆₋₂₀ fatty acid glycerides, for example glyceryl monostearate, glyceryl distearate, glyceryl laurate, glyceryl dilaurate, glyceryl palmitate, glyceryl dipalmitate.

The oil phase in the emulsions may also comprise alkyl esters of fatty acids, wherein the alkyl group in the latter may be linear or branch chained, saturated or unsaturated, substituted or unsubstituted, and may have from 1 to 30 carbon atoms, in particular from 1 to 22 carbon atoms. The fatty acids in said alkyl esters may be linear or branch chained, saturated or unsaturated, substituted or unsubstituted C₆₋₃₀ fatty acids or fatty acid mixtures, in particular C₆₋₂₄ fatty acids, more in particular C₈₋₂₂ fatty acids, preferably C₁₂₋₁₈ fatty acids. The alkyl groups in said esters are derived preferably from fatty alcohols as well as of mixtures thereof, in particular C₁₋₂₄ fatty alcohols, more in particular C₆₋₂₂ fatty alcohols, preferably C₈₋₁₈ fatty alcohols. Fatty alcohol mixtures for use in these esters for example, may be obtained by high-pressure hydrogenation of technical mixtures of the methyl esters derived from fats or oils.

Examples of oil components of the ester type are the following: decyl oleate, coco caprylate/-caprate, hexyl laurate, myristyl isostearate, myristyl oleate, cetyl isostearate, cetyl oleate, cetyl stearate, cetyl behenate, cetyl palmitate, cetearyl behenate, isostearyl myristate, isostearyl palmitate, isostearyl stearate, isostearyl isostearate, isostearyl oleate, isostearyl behenate, oleyl myristate, oleyl isostearate, oleyl oleate, oleyl erucate, oleyl palmitate, oleyl stearate, oleyl isostearate, oleyl oleate, oleyl behenate, erucyl isostearate, erucyl oleate, palmityl stearate, stearyl heptanoate, stearyl octanoate, stearyl stearate, stearyl isostearate, stearyl behenate, stearyl oleate, myristyl myristate, myristyl palmitate, myristyl stearate, myristyl isostearate, myristyl oleate, behenyl behenate, behenyl isostearate, behenyl oleate,

Interesting oil components of the ester type are the esters from linear C₆₋₂₂-fatty acids with branched C₁₋₁₂ alkanols, in particular with 2-ethylhexanol (e.g. the stearate of the latter commercially available under the trade name Cetiol™ 868); esters from branched C₆₋₂₂-fatty acids with linear C₁₋₁₂ alkanols; esters from C₁₂₋₃₀-hydroxy fatty acids with linear or branched C₆₋₂₂-fatty alcohols; esters from linear or branched fatty acids with multifunctional alcohols (e.g. propylene glycol, dimerdiol oder trimertriol) or with Guerbet alcohols; esters from C₆₋₂₂-fatty alcohols or Guerbet alcohols with aromatic carboxylic acids, in particular with benzoic acids; esters from C₂-C₁₂-dicarboxylic acids with linear or branched alcohols having 1 to 22 carbon atoms (e.g. dioctyl maleates); esters of linear or branched fatty acids with poly-alcohols (e.g. propylene glycol, dimerdiol or trimertriol) or with Guerbet alcohols. As used herein the Guerbet alcohols may be based on fatty alcohols having 6 to 18, in particular 8 to 10 carbon atoms.

Other oil components that may be used comprise fatty alcohols in particular any saturated or unsaturated, straight or branch chained fatty alcohols, including mixtures thereof. Fatty alcohols comprise, for example, C₁₂-C₅₀-fatty alcohols, in particular the C₁₂-C₂₄-fatty alcohols, more in particular the C₁₆-C₂₂-fatty alcohols that are derived from natural fats, oils or waxes such as, for example, myristyl alcohol, 1-pentadecanol, cetylalcohol, 1-heptadecanol, stearyl alcohol, 1-nonadecanol, arachidyl alcohol, 1-heneicosanol, behenyl alcohol, brassidyl alcohol, lignoceryl alcohol, ceryl alcohol, myricyl alcohol, lauryl alcohol, capryl alcohol, caprinyl alcohol, cetyl alcohol, palmoleyl alcohol, isostearyl alcohol, oleyl alcohol, elaidyl alcohol, petroselinyl alcohol, arachidyl alcohol, gadoleyl alcohol, erucyl alcohol, including mixtures thereof such as cetearyl alcohol, C_{12/13} fatty alcohol, as well as Guerbet alcohols.

Mixtures of fatty alcohols can evidently also be used, including fatty alcohol fractions obtained from the reduction of the corresponding fatty acid fractions derived from naturally occurring oils or fats such as the natural oils or fats mentioned above.

Further oil components comprise fatty acids in particular C₁₄-C₄₀-fatty acids, and hydroxy fatty alcohols, in particular C₁₂-C₃₀-hydroxy fatty alcohols. Other oil components, which can be used comprise mineral and paraffin oils and synthetic oils, either aliphatic or aromatic, as well as mixtures thereof.

Further oil components that can be added are the Guerbet alcohols which may be based on fatty alcohols having 6 to 18, in particular 8 to 10 carbon atoms.

Still further oil components that are hydrocarbons comprise, for example, squalane, squalene, paraffine oils, isohexadecane, isoeicosane or polydecene as well as dialkylcyclohexanes.

Still further oil components are silicone oils such as, for example cyclic silicones, dialkyl- or alkylarylsiloxanes, e.g., cyclomethicone, dimethyl polysiloxane and methylphenyl polysiloxane, as well as the alkoxylated and quaternized analogs thereof. Appropriate non-volatile silicone oils are e.g. polyalkylsiloxanes, polyalkylarylsiloxanes and polyethersiloxane-copolymers. A particularly suitable silicone oil can be a dimethylpolysiloxane having trimethylsiloxy groups at both molecular terminals, a methylphenylpolysiloxane having trimethylsiloxy groups at both molecular terminals, a copolymer of methylphenylsiloxane and dimethylsiloxane having trimethylsiloxy groups at both molecular terminals, a cyclic dimethylsiloxane, or a cyclic methylphenylsiloxane.

Preferred silicone oils comprise cyclic dimethylsilicones, i.e. the cyclomethicones, e.g. tetracyclomethicone, pentacyclomethicone, and linear dimethylsilicones, i.e. the dimethicones, including any mixture of these.

The oil phase may also contain certain amounts of solid or semi-solid lipophilic components such as fats and waxes, which are materials that are liquid above ambient temperature, in particular above 25 °C. Fats comprise triglyceride materials from natural origin or synthetic. Waxes comprise synthetic or natural waxes. The latter comprise Japan wax, Ouricury wax, montan wax, castor wax, carnauba wax, candelilla wax, beeswax, ceresin wax and ozokerite waxes. Synthetic waxes comprise petroleum-based waxes such as paraffin, petrolatum, polyalkylene glycol waxes, e.g. polyethylene wax.

Waxes also encompass materials, which are the higher homologues of the materials mentioned hereinabove as oils, for example the higher esters, the higher mono-, di- and triglycerides, the higher alkanols (in particular the higher fatty alcohols), higher alkanediols (in particular hydroxy fatty alcohols), the higher fatty acids. As further useful components there can be mentioned silicone waxes.

### The aqueous phase

The water phase in the O/W emulsions of this invention may be pure water but usually contains one or more hydrophilic components. The latter can be lower alkanols, polyols, water-soluble active ingredients, preservatives and moisturizers, chelating agents, etc.

### Emulsifier

The O/W emulsions of this invention additionally contain one or more emulsifiers suitable for making oil-in-water emulsions. These emulsifiers may be anionic, cationic, amphoteric or non-ionic emulsifiers, including mixtures thereof. Preferred are non-ionic emulsifiers.

Suitable anionic emulsifiers include the fatty acid soaps, e.g. sodium, potassium and triethanolamine soaps, wherein the fatty acid moiety may contain from 10 to 20 carbon atoms. Other suitable anionic emulsifiers include the alkyl sulfonates. alkyl sulfates, alkylaryl sulfonates, alkyl polyethoxy ether sulfonates, alkylphenol polyethoxy ether sulfonates, mono- or diesters of sulfosuccinic acid, in particular the alkali metal, ammonium or substituted ammonium salts thereof. The alkyl group may 10 to 30 carbon atoms and the polyethoxylated anionic emulsifiers may contain from 1 to 50 and in particular 2 to 25 ethylene oxide units.

Suitable cationic emulsifiers are quaternary ammonium, morpholinium and pyridinium salts, in particular the tetraalkylammonium halides such as the alkyltrimethylammonium halides, wherein the alkyl group may 10 to 30 carbon atoms.

Particular non-ionic emulsifiers comprise :
(1) Addition products of 2 to 50 moles of ethylene oxide and/or 0 to 20 moles propylene oxide to linear fatty alcohols having 8 to 40 C-atoms, to fatty acids with 12 to 40 C-atoms and to alkylphenols with 8 to 15 C-atoms in the alkyl rest.
(2) C_{12/18}-fatty acid mono- and -diesters of addition products of 1 to 50 moles of ethylene oxide and glycerine.
(3) Glycerine mono- and -diesters and sorbitan mono- and -diesters of saturated and unsaturated fatty acids with 6 to 22 C-atoms and their ethylene oxide addition products.
(4) Alkyl mono- and -oligoglycosides with 8 to 22 C-atoms in the alkyl rest and the ethoxylated analogs thereof.
(5) Addition products of 7 to 60 moles of ethylene oxide to castor oil and/or hardened castor oil.
(6) Polyol- and in particular polyglycerine esters, such as e.g. polyol poly-12-hydroxystearate, polyglycerine polyricinoleate, polyglycerine diisostearate or polyglycerine dimerate. Also applicable are mixtures of compounds of several of these substance classes.
(7) Addition products of 2 to 15 moles of ethylene oxide to castor oil and/or hardened castor oil.
(8) Partial esters derived from linear, branch chained, unsaturated or saturated C₆-C₂₂-fatty acids, ricinoleic acid as well as 12-hydroxystearic acid and glycerine, polyglycerine, pentaerythrite, dipentaerythrit, sugar alcohols (e.g. sorbitol), alkylglucosides (e.g. methylglucoside, butylglucoside, laurylglucoside) as well as polyglucosides (e.g. cellulose), or mixed esters such as e.g. glyceryl stearate/citrate and glyceryl stearate/lactate.
(9) Wool wax alcohols.
(10) Polysiloxane-polyalkyl-polyether-copolymers and derivatives thereof.
(11) Mixed esters from pentaerythrite, fatty acids, citric acid and fatty alcohols and/or mixed esters of fatty acids with 6 to 22 C-atoms with methylglucose and polyoles, respectively glycerine or polyglycerine.
(12) Polyalkylene glycols, including block copolymers of ethylene oxide and propylene oxide.

The addition products of ethylene oxide and/or of propylene oxide and fatty alcohols, fatty acids, alkylphenoles, glycerine mono- and -diesters as well as sorbitan mono- and -diesters of fatty acids or of castor oil are known and commercially available products. Usually these are mixtures of homologues of which the average degree of alkoxylation corresponds to the ratio of starting quantities of ethylene oxide and/or propylene oxide and substrate, with which the addition reaction is conducted.

Examples of suitable zwitterionic emulsifiers include alkyldimethylamine oxides, the alkyl group having from 6 to 16 carbon atoms.

The emulsions may comprise from about 1% to 10%, preferably from about 2% to 5%, of emulsifier.

### Active ingredients

The emulsions of the invention may contain active ingredients for application to the skin. Examples of active agents comprise anti-microbial agents, e.g. anti-bacterials and antifungals, anti-inflammatory agents, anti-irritating compounds, anti-itching agents, moisturising agents, skin caring ingredients, plant extracts, vitamins, and the like. Examples of such ingredients comprise complexes of PVP and hydrogen peroxide, anti-inflammatories as, plant extracts, bisabolol, panthenol, tocopherol, actives for anti-stinging, anti-irritants, anti-dandruffs, for anti-ageing e.g. retinol, melibiose etc. Other suitable actives are e.g. allantoin, Aloe barbadensis, Avena sativa, beta-carotene, bisabolol, Borago officinalis, Chamomilla recutita, dichlorophenyl imidazoldioxolan, ethyl panthenol, famesol, ferulic acid, Ginkgo biloba, heliotropine, hydrolyzed wheat protein, Juniperus communis, lactis proteinum, lactose,, linalool, lysine, mannitol, Mentha piperita, menthol, menthyl lactate, olaflur, Oryza sativa, panthenol, Prunus amygdalus dulcis, retinyl palmitate, Ricinus communis, Rosmarinus officinalis, salicylic acid, sodium carboxymethyl betaglucan, sodium cocoyl amino acids, sodium hyaluronate, sodium palmitoyl proline, thymus vulgaris, tocopherol, tocopheryl acetate, tyrosine, valine, etc.

The active ingredients can be present, depending on the nature of the ingredients and their application, in various concentrations, but usually are present in a quantity in the range of from 0.01 - 10 % (w/w), preferably from 0.1-7 % (w/w) and more preferably from 1 -5 % (w/w) expressed to the total weight of the emulsion.

The emulsions according to this invention can be used as anti-perspirants or deodorants wherein one or both phases of the emulsion contain actives that have deodorizing and /or anti-perspirant properties. Actives that can be used to this purpose are anti-perspirant agents such as, for example, aluminium chlorohydrates, aluminium-zirconium-chlorohydrate as well as zinc salts. Other such agents comprise aluminium hydroxylactates as well as acid aluminium/zirconium salts. A particularly suitable chlorohydrate is the compound of formula [Al₂(OH)₅Cl]·2.5 H₂O. Further such agents are aluminium-zirconium-tetrachlorohydroxy-glycine-complexes. Esterase inhibitors can be added as further deodorizing agents, i.e. agents such as trialkyl citrates such as trimethylcitrate, tripropyl citrate, triisopropyl citrate, tributyl citrate and in particular triethyl citrate. Further esterase inhibitors are sterol sulfates or -phosphates, such as, for example, lanosterine-, cholesterine-, campesterine-, stigmasterine- and sitosterine sulfate respectively -phosphate, dicarboxylic acids and their esters, such as, for example, glutaric acid, glutaric acid monoethylester, glutaric acid diethylester, adipinic acid, adipinic acid monoethylester, adipinic acid diethylester, malonic acid and malonic acid diethylester, hydroxycarbonic acids and their esters such as, for example, citric acid, malonic acid, tartaric acid or tartaric acid diethylester.

Antibacterial active ingredients that influence the growing conditions and eradicate perspiration decomposing bacteria, or impede their growth, can also be present in the lipid and/or aqueous phase. Examples of such ingredients are phenoxyethanol, chlorohexidine gluconate and 5-chloro-2-(2,4-dichlorophenoxy)-phenol.

The emulsions according to the invention can also be used in sunscreen applications and in that instance take the form of sunscreen sprays. In these products, the lipid and/or aqueous phase contains one or more sunscreen filters which are for example organic substances that are capable of absorbing ultraviolet radiation and to set free the absorbed energy as longer-wave radiation.

UVB-filters can be oil or water-soluble. As oil-soluble substances there can be mentioned for example:
- 3-Benzylidene campher respectively 3-benzylidene norcampher and derivatives thereof, e.g. 3-(4-methylbenzylidene) campher;
- 4-Aminobenzoic acid derivatives, respectively 4-(dimethylamino)benzoic acid-2-ethylhexyl esters, 4-(dimethylamino)benzoic acid-2-octyl esters and 4-(dimethylamino)benzoic acid amylesters;
- Esters of cinnamonic acid, in particular 4-methoxycinnamonic acid-2-ethylhexylester, 4-methoxycinnamonicacid propylester, 4-methoxycinnamonic acid isoamyl ester, 2-cyano-3,3-phenylcinnamonic acid-2-ethylhexyl ester (octocrylene);
- Esters of salicylic acid, respectively salicylic acid-2-ethylhexylester, salicylic acid-4-isopropylbenzyl ester, salicylic acid homomenthyl ester;
- Derivatives of benzophenones, in particular 2-hydroxy-4-methoxybenzophenone, 2-hydroxy-4-methoxy-4'-methylbenzophenone, 2,2'-dihydroxy-4-methoxybenzophenone;
- Esters of benzalmalonic acid, in particular 4-methoxybenzmalonic acid di-2-ethylhexyl ester;
- Triazine derivatives, such as, for example, 2,4,6-trianilino-(p-carbo-2' -ethyl-1'-hexyloxy)-1,3,5-triazine and octyltriazone;
- Propane-1,3-diones, such as for example, 1-(4-tert.butylphenyl)-3-(4'-methoxyphenyl)propane-1,3-dione;
- Ketotricyclo(5.2.1.0)decane-derivatives.

Water-soluble UV-filter are for example:
- 2-Phenylbenzimidazol-5-sulfonic acid and its alkali-, alkaline earth-, ammonium-, alkylammonium-, alkanolammonium- and glucammonium salts;
- Sulfonic acid derivatives of benzophenones, in particular 2-hydroxy-4-methoxybenzophenone-5-sulfonic acid and its salts;
- Sulfonic acid derivatives of 3-benzylidene campher, e.g. 4-(2-oxo-3-bomylidene methyl)benzol-sulfonic acid and 2-methyl-5-(2-oxo-3-bomylidene)sulfonic acid and its salts.

Typical UV-A-Filters that can be used are derivatives of benzoylmethane, such as, for example, 1-(4'-tert.butylphenyl)-3-(4'-methoxyphenyl)propane-1,3-dione, 4-tert.-butyl-4'-methoxydibenzoylmethane (Parsol™ 1789), or 1-phenyl-3-(4'-isopropylphenyl)-propane-1,3-dione. Mixtures of UV-A and UV-B-filters can be evidently used too.

Of particular interest are the so-called broadband filters. One type of such filters are the water-soluble filters, more specifically the benzotriazoles, in particular the benzotriazole derivate known as 2,2'-methylene-bis- (6-(2H- benzotriazole-2-yl)-4-(1,1,3,3-tetramethylbutyl)-phenol) [INCI: Bisoctyltriazol], which is commercially available under the tradename Tinosorb™ M from CIBA Chemicals. Another useful benzotriazole derivate is 2-(2H-benzotriazole-2-yl)-4-methyl-6-[2-methyl-3-[1,3,3,3-tetramethyl-1-[(trimethylsilyl)oxy]disiloxanyl]propyl]-phenol (CAS-No.: 155633-54-8) also indicated by the INCI name drometrizole trisiloxane and is available from Chimex under the tradename Mexoryl™ XL. These benzotriazole derivatives can be conveniently incorporated in the water phase at a pH >4.5.

Other useful water-soluble UV filters are the sulfonated UV filters such as 3,3'-(1,4-phenylenedimethylene) bis (7,7-dimethyl-2-oxo-bicyclo-[2.2.1]hept-1- yl methanesulfonic acid, and its sodium, potassium or its triethanolammonium salt, and the sulfonic acid itself, indicated by the INCI name terephthalidene dicamphor sulfonic acid (CAS No. 90457- 82-2), which is available, for example, under the trade name Mexoryl™ SX from Chimex.

Oil-soluble broadband filters are the asymmetrically substituted triazine derivatives. Of particular interest is 2,4- Bis-{[4-(2-ethyl-hexyloxy)-2-hydroxy]-phenyl}-6-(4-methoxyphenyl)-1,3,5-triazine (INCI: anisotriazine), that is commercially available under the tradename Tinosorb™ S from CIBA Chemicals.

A further advantageous water-soluble UV filter is 3,3'-(1,4- phenylenedimethylene) bis (7,7-dimethyl-2-oxo-bicyclo-[2.2.1]hept-1-ylmethanesulfonic acid, as well its sodium, potassium or its triethanolammonium salt, with the INCI name terephthalidene dicamphor sulfonic acid (CAS No. 90457- 82-2), which is available, for example, under the trade name Mexoryl® SX from Chimex.

Specific embodiments in accordance with this invention are sprayable oil-in-water emulsions, which are sunscreen formulations, having an SPF ≥40, in particular ≥50, or ≥60, comprising an oil-in-water emulsion wherein the emulsion contains from 15% to 40%, in particular from 20 to 30% of an oil phase, said emulsions comprising:
(a) from 5 to 30%, in particular from 10 to 30%, more in particular from 10 to 30%, further in particular from 10 to 20% of lipophilic UV filters, which filters are included in the oil phase;
(b) from 0.1 to 10%, in particular from 0.2 to 5%, more in particular from 0.3 to 3% of an alkylglycol, preferably caprylyl glycol.

Further embodiments of this invention are sprayable oil-in-water emulsions, which are sunscreen formulations, having an SPF ≥40, in particular ≥50, or ≥60, wherein the emulsion contains from 15 % to 40%, in particular from 20 to 30 % of an oil phase, comprising :
(a) from 5 to 30 %, in particular from 10 to 30%, more in particular from 10 to 30%, further in particular from 10 to 20% of lipophilic UV filters, which filters are included in the oil phase;
(b) from 1 to 10%, in particular from 1 to 5%, more in particular from 2 to 5% of hydrophilic UV filters;
(c) from 0.1 to 10%, in particular from 0.2 to 5%, more in particular from 0.3 to 3% of an alkylglycol, preferably caprylyl glycol at least 0.3 % of an alkylglycol.

Further embodiments of this invention are sprayable oil-in-water emulsions, which are sunscreen formulations, having an SPF ≥40, in particular ≥50, wherein the emulsion contains from 15 % to 40%, in particular from 20 to 30 % of an oil phase, said emulsions comprising :
(a) from 5 to 30 %, in particular from 10 to 30%, more in particular from 10 to 30%, further in particular from 10 to 20% of lipophilic UV filters, which filters are included in the oil phase;
(b) from 1 to 10%, in particular from 1 to 5%, more in particular from 2 to 5% of hydrophilic UV filters;
(c) from 0.5% to 15%, in particular from 1 to 10%, more in particular from 1 to 5% of an inorganic pigment, in particular titanium dioxide or coated titanium dioxide;
(d) from 0.1 to 10%, in particular from 0.2 to 5%, more in particular from 0.3 to 3% of an alkylglycol, preferably caprylyl glycol.

Further embodiments of this invention are sprayable sunscreen formulations having an SPF ≥40, in particular ≥50, comprising an oil-in-water emulsion wherein the emulsion contains from 15 % to 40%, in particular from 20 to 30 % of an oil phase, comprising:
(a) from 5 to 30 %, in particular from 10 to 30%, more in particular from 10 to 30%, further in particular from 10 to 20% of lipophilic UV filters, which filters are included in the oil phase;
(b) from 1 to 10%, in particular from 1 to 5%, more in particular from 2 to 5% of hydrophilic UV filters;
(c) from 0.5% to 15%, in particular from 1 to 10%, more in particular from 1 to 5% of an inorganic pigment, in particular titanium dioxide or coated titanium dioxide;
(d) from 0.1 to 15%, in particular from 0.5% to 10%, more in particular from 1 to 10% of latex particles, in particular of hollow latex particles;
(e) from 0.1 to 10%, in particular from 0.2 to 5%, more in particular from 0.3 to 3% of an alkylglycol, preferably caprylyl glycol.

Spray formulations having an SPF ≥40, in particular ≥50 preferably contain both a benzotriazole and an asymmetrically substituted triazine derivative, in particular both the components Tinosorb™ M and Tinosorb™ S.

Apart from the above-mentioned soluble UV filter substances, there can also be added insoluble sunscreen pigments, namely finely dispersed metal oxides or metal salts. Examples of appropriate metal oxides in particular are zinc oxide and titanium dioxide as well as oxides of iron, zirconium, silicon, manganese, aluminium and cerium as well as mixtures thereof. Salts that can be used comprise silicates (talcum), barium sulfate or zinc stearate. The particle size of these pigments is sufficiently small, e.g. less than 100 nm, in particular between 5 and 50 nm and more in particular between 15 and 30 nm. The particles can be spherical but can have other shapes too such as ellipsoidal or similar shapes. The surface of the pigments may have been treated, e.g. hydrophilized or made hydrophobic. Typical examples are coated titanium dioxide, e.g. Titanium dioxide T 805 (available from Degussa) or Eusolex® T 2000 (Merck). Silicones can be used as hydrophobic coating agents, in particular trialkoxyoctyl silanes or simethicones. So-called micro- or nanopigments are particularly attractive for use in sunscreen products.

Other components that can be added are latex particles, in particular latex particles having a mean particle size of 100 to 400 nm. Of particular interest are the hollow latex particles described in US-5,663,213 corresponding to EP-A-0 761 201. Particularly preferred are those latex particles that are made of styrene/acrylate copolymers and which are available under the tradename 'Alliance SunSphere™^{,} from Rohm & Haas. These commercial products usually have a content of 'active' (i.e. of pure latex) of about 25 to 27 weight-% and when used in the emulsions of this invention the quantity of commercial product needs to be recalculated in function of the quantity of the latex particles present therein.

Apart from both groups of primary light protecting filters that are mentioned above there can also be used secondary light protecting factors. These pertain to the class of anti-oxidants and their activity is based on the interruption or decrease of the photochemical processes caused by solar radiation upon penetration in the skin.

Typical examples of secondary light protecting agents are amino acids such as for example glycine, histidine, tyrosine and tryptophane, including derivatives of amino acids; imidazoles (for example urocanic acid) and derivatives thereof; peptides such as D,L-camosine, D-camosine, L-camosine and derivatives thereof (e.g. anserin). Further agents that can be used are carotinoides, carotenes (for example α-carotene, β-Carotene and lycopene) and derivatives thereof; chlorogenic acid and its derivatives; lipoic acid and derivatives thereof (e.g. dihydrolipoic acid), aurothioglucose, propylthiouracil and other thioles (for example thioredoxine, glutathione, cysteine, cystamine and glycosyl-, N-acetyl-, methyl-, ethyl-, propyl-, amyl-, butyl- and lauryl-, palmitoyl-, oleyl-, γ-linoleyl-, cholesteryl- and glyceryl esters) and their salts. Further examples are dilauryl thiodipropionate, distearyl thiopropionate, thiodipropionic acid and derivatives thereof (for example esters, ethers, peptides, lipids, nucleotides, nucleosides and salts), sulfoximine compounds (for example buthionine sulfoximine, homocysteine sulfoximine, butionine sulfone, penta- hexa-, and heptathione sulfoximine). These secondary agents usually are formulated at very low concentrations (e.g. pmol to µmol/kg),

Other secondary agents (usually in small concentrations, as mentioned above) are chelating agents (for example α-hydroxy fatty acids, palmeate acid, phytic acid, lactoferrin), α-hydroxy acids (for example citric acid, lactic acid, malic acid), humic acid, bile acid, bile extracts, bilirubin, biliverdin, EDTA, EGTA and derivatives thereof; unsaturated fatty acids and derivatives thereof such as for example γ-linolenic acid, linoleic acid, oleic acid, folic acid and derivatives thereof, ubiquinones and ubiquinol and derivatives thereof, vitamin C and derivatives thereof (e.g. ascorbyl palmitate, Mg ascorbylphosphate, ascorbyl acetate), tocopherol and derivatives thereof (for example vitamin E acetate), vitamin A and derivatives thereof (e.g. vitamin A palmitate), coniferyl benzoates of benzoic acid, rutinic acid and derivatives thereof, α-glycosyl rutin, ferula acid, furfurylidene glucitol, carnosine, butyl hydroxytoluene, butyl hydroxyanisol, nordihydroguaiac resin acid, nordihydroguaiaret acid, trihydroxybutyrophenone, ureic acid and derivatives thereof, mannose and derivatives thereof, superoxide-dismutase, zinc and derivatives thereof such as zinc oxide, zinc sulphate, selenium and derivatives thereof (e.g. seleno-methionine); stilbene and derivatives thereof (e.g. stilbene oxide, trans stilbene oxide); and any appropriate derivatives of these UV filters.

The sprayable emulsions of this invention can also be used as self-tanning sprays. In that instance there can be added dihydroxy acetone.

The lipid and/or aqueous phase can further contain one or more moisturizers, which may be present in quantities of 1 -20 % (w/w), preferably of 5 -15 % (w/w), and more preferably 5 -10 % (w/w) - relative to the total amount of the emulsion.

Suitable moisturizers are a.o. amino acids, pyrrolidone carbonic acid, lactic acid and its salts, lactitol, urea and urea derivatives, ureic acid, glucosamine, creatinine, hydrolysis products of collagen, chitosan or chitosan salts/-derivatives , and in particular polyols and polyol derivatives such as ethylene glycol, propylene glycol, butylene glycol, pentylene glycol, hexylene glycol, erythrite, 1,2,6-hexanetriol, polyethylene glycols such as PEG-4, PEG-6, PEG-8, PEG-10, PEG-12, PEG-14, PEG-16, PEG-18, PEG-20, PEG-135, PEG-150, sugar and sugar derivatives (a.o. fructose, glucose, maltose, maltitol, mannite, inosite, sorbite, sorbityl silandiol, sucrose, trehalose, xylose, xylite, glucuronic acid and its salts), ethoxylated sorbitol, honey and hydrogenated honey, hydrogenated starch hydrolysates, as well as mixtures of hydrogenated wheat protein, hydrolyzed milk protein, lecithin, pythantriol, hyaluronic acid and salts thereof, and PEG-20-acetate copolymers. Particularly preferred moisturizers are glycerine, diglycerine and triglycerine.

The emulsions of the invention may contain perfume oils which can be mixtures of synthetic or natural odorous substances extracted from blossoms (lilly, lavender, rose, jasmine, neroli, ylang-ylang), from stems and leaves (geranium, patchouli, petitgrain), from fruits (anis, coriander, caraway, juniper), from cortex (bergamot, lemon, orange), from roots (macis, angelic, celery, cardamon, costus, iris, calmus), from wood (pine, sandelwood, guajak, cedar, rose), from herbs and grass (tarragon, lemongrass, sage, thyme), from needles and branches (spruce, fir, pine, mountain pine), from resins and balms (galbanum, elemi, benzoine, myrrh, olibanum).

The emulsions of this invention may contain one or more preservatives. Preservatives are useful for substantially preventing microbial decomposition. Examples of preservatives include phenoxyethanol, iodopropyl butylcarbamate (IPBC), sodium benzoate, and parabens such as methyl-paraben, ethylparaben, and propylparaben. Other examples of preservatives are listed on pages 1654- 55 of the International Cosmetic Ingredient Dictionary and Handbook, eds. Wenninger and McEwen (CTFA, 7th ed., 1997), hereinafter referred to as the "Cosmetic Handbook." The compositions may comprise from about 0.01 % to about 10%, by weight (more preferably, from about 0.5% to about 5%, by weight) of preservative.

Antioxidants and/or chelating agents may also be used to increase shelf life and stability of the compositions. Antioxidant compounds and their derivatives include, but are not limited to, water-soluble antioxidants such as sulfhydryl compounds and their derivatives (e.g., sodium metabisulfite and N-acetyl-cystein), lipoic acid and dihydrolipoic acid, resveratrol, acetyl-cysteine or lactoferrin, ascorbic acid and ascorbic acid derivatives (e.g., ascorbyl palmitate and ascorbyl polypeptide). Oil-soluble antioxidants include, but are not limited to, butylated hydroxytoluene, retinoids (e.g. retinol and retinyl palmitate), tocopherols (e.g., tocopherol acetate), tocotrienols, and ubiquinone. Natural extracts containing antioxidants comprise extracts containing flavonoids and isoflavonoids and their derivatives (e.g., genistein and diadzein), extracts containing resveratrol and the like. Examples of such natural extracts include grape seed, green tea, pine bark, propolis, and legume extracts. Other examples of antioxidants may be found on pages 1612-13 of the Cosmetic Handbook. The compositions of the present invention may comprise the antioxidant in an amount of from about 0.001% to about 20%, by weight (e.g., from about 0.01% to about 10% by weight) of the composition.

Chelating agents may also be added for example to assist the stabilization of the emulsions of this invention. Examples of chelating agents include EDTA and derivatives thereof (e.g., disodium EDTA and dipotassium EDTA), Iniferine ™, lactoferrin, and citric acid. Other examples of chelating agents are listed on page 1626 of the Cosmetic Handbook. The emulsions of the present invention may comprise the chelating agent in an amount of from about 0.001% to about 20%, by weight (e.g., from about 0. 01% to about 10% by weight) of the composition.

The emulsions of the invention are prepared following art-known procedures to prepare oil-in-water emulsions. In general, the oil and the water phases are prepared separately whereupon the oil phase is added to the water phase or vice versa, upon vigorous stirring as to form the emulsion. If desired, additional components may be added to the emulsion. If desired, the emulsification process may be done at increased temperature, e.g. at a temperature above 50 °C, or above 70 °C.

The sprayable emulsions of this invention may find use in instances where high lipid content is required, e.g. emulsions with relatively high amounts of active ingredients that are lipophilic, e.g. sunscreen formulations with high SPF, or sprays aimed at delivering oils.

The following examples are given to illustrate the invention and not to limit it thereto.

### Examples

### Example 1

### Sprayable sunscreen formulation of high SPF (SPF ≥50)

| INCI Name | % (w/w) |
|---|---|
| Aqua | 59.475 |
| Ethylhexyl Methoxycinnamate | 7.50 |
| 4-Methylbenzylidene Camphor | 4.00 |
| Butylene Glycol | 3.00 |
| PVP/Hexadecene Copolymer | 3.00 |
| C12-15 Alkyl Benzoate | 3.00 |
| Methylene Bis-Benzotriazolyl Tetramethylbutylphenol 2.5% / Decyl Glucoside 0.375% | 2.875 |
| Styrene/Acrylates Copolymer | 2.50 |
| Bis-Ethylhexyloxyphenol Methoxyphenyl Triazine | 2.00 |
| Dimethicone | 2.08 |
| Glycerine | 2.00 |
| Sodium benzoate | 1.82 |
| Titanium Dioxide coated with aluminium hydroxide/stearic acid (5.7 : 1 : 1 mixture) | 1.50 |
| Hydrogenated Palm Glycerides Citrate | 0.034 |
| Carbomer (0.10) /water (0.11) | 0.21 |
| PEG-100 Stearate | 0.75 |
| Glyceryl Stearate | 0.75 |
| Potassium Cetyl Phosphate | 0.80 |
| Cetyl Alcohol | 0.50 |
| Caprylyl Glycol | 0.50 |
| PEG-4 | 0.008 |
| Pentylene Glycol | 0.55 |
| PEG-4 Laurate | 0.032 |
| PEG-4 Dilaurate | 0.032 |
| Linseed Acid | 0.01 |
| Sodium Dodecylbenzenesulfonate | 0.01 |
| Disodium EDTA | 0.10 |
| Tocopherol | 0.116 |
| Sodium Hydroxide | 0.04 |
| Phenoxyethanol | 0.80 |
| Iodopropynyl Butylcarbamate | 0.008 |
| TOTAL | 100.00 |

## Claims

1. A sprayable oil-in-water emulsion comprising at least 15% of oil phase, wherein the formulation contains at least 0.1 % of an alkylglycol.

2. A sprayable oil-in-water emulsion according to claim 1, comprising at least 5% of lipophilic active ingredients.

3. A sprayable oil-in-water emulsion according to claim 2 which is a sprayable sunscreen formulation, comprising at least 5 % of lipophilic UV filters and wherein the emulsion contains at least 15 % of an oil phase, wherein the formulation contains at least 0.3 % of an alkylglycol.

4. A sprayable oil-in-water emulsion according to claim 3, having an SPF ≥ 40, more preferably ≥ 50, or even ≥ 60.

5. A sprayable oil-in-water emulsion according to any of claims 1-4 wherein the emulsion contains at least 20%, more in particular at least 25 %, or further in particular at least 30 % of lipid phase.

6. A sprayable oil-in-water emulsion according to any of claims 1-5 wherein the emulsion contains at least 0.3 % of alkyl glycol, more in particular at least 0.3 % of alkyl glycol.

7. A sprayable oil-in-water emulsion according to claim 1, wherein the emulsion contains from 15 % to 40%, in particular from 20 to 30 % of an oil phase, and further comprising from 0.1 to 10%, in particular from 0.2 to 5%, more in particular from 0.3 to 3% of an alkylglycol.

8. A sprayable oil-in-water emulsion according to claim 1, wherein the emulsion contains from 15 % to 40%, in particular from 20 to 30 % of an oil phase, said emulsion comprising :
(a) from 5 to 30 %, in particular from 10 to 30%, more in particular from 10 to 30%, further in particular from 10 to 20% of one or more lipophilic active ingredients, which active ingredients are included in the oil phase;
(b) from 0.1 to 10%, in particular from 0.2 to 5%, more in particular from 0.3 to 3% of an alkylglycol, preferably caprylyl glycol.

9. A sprayable oil-in-water emulsion according to claim 1, which is a sunscreen formulation, wherein the emulsion contains from 15 % to 40%, in particular from 20 to 30 % of an oil phase, said emulsion comprising :
(a) from 5 to 30 %, in particular from 10 to 30%, more in particular from 10 to 30%, further in particular from 10 to 20% of lipophilic UV filters, which filters are included in the oil phase;
(b) from 0.1 to 10%, in particular from 0.2 to 5%, more in particular from 0.3 to 3% of an alkylglycol, preferably caprylyl glycol.

10. A sprayable oil-in-water emulsion according to any of claims 1 - 9 wherein the alkyl glycol is caprylyl glycol.

11. A sprayable oil-in-water emulsion according to claim 1, having an SPF ≥ 40, in particular ≥ 50, wherein the emulsion contains from 15 % to 40%, in particular from 20 to 30 % of an oil phase, said emulsion comprising:
(a) from 5 to 30 %, in particular from 10 to 30%, more in particular from 10 to 30%, further in particular from 10 to 20% of lipophilic UV filters, which filters are included in the oil phase;
(b) from 1 to 10%, in particular from 1 to 5%, more in particular from 2 to 5% of hydrophilic UV filters;
(c) from 0.1 to 10%, in particular from 0.2 to 5%, more in particular from 0.3 to 3% of an alkylglycol, preferably caprylyl glycol at least 0.3 % of an alkylglycol.

12. A sprayable oil-in-water emulsion according to claim 11, said emulsion comprising:
(a) from 5 to 30 %, in particular from 10 to 30%, more in particular from 10 to 30%, further in particular from 10 to 20% of lipophilic UV filters, which filters are included in the oil phase;
(b) from 1 to 10%, in particular from 1 to 5%, more in particular from 2 to 5% of hydrophilic UV filters;
(c) from 0.5% to 15%, in particular from 1 to 10%, more in particular from 1 to 5% of an inorganic pigment, in particular titanium dioxide or coated titanium dioxide;
(d) from 0.1 to 10%, in particular from 0.2 to 5%, more in particular from 0.3 to 3% of an alkylglycol, preferably caprylyl glycol.
